# EUROPEAN PATENT SPECIFICATION

(11) **EP 1 141 022 B1**
(45) Date of publication and mention of the grant of the patent: **27.10.2004**
(21) Application number: 99962602.1
(22) Date of filing: 23.12.1999
(51) Int. Cl.: C07K 14/81, C07H 21/04, C12N 15/66, C12N 15/70, C12N 15/74, C12N 15/79, C12N 15/81, C12N 1/21, C12N 1/19, A61K 38/55, A61K 38/57, A61P 7/02, A23J 1/04

(54) **SERINE PROTEASE INHIBITOR**
SERINPROTEASE-INHIBITOR
INHIBITEUR DE SERINE PROTEASE

(30) Priority: 23.12.1998 NZ 33356898; 23.07.1999 NZ 33690699
(43) Date of publication of application: 10.10.2001
(73) Proprietor: THE HORTICULTURE AND FOOD RESEARCH INSTITUTE OF NEW ZEALAND LIMITED, Palmerston North 5331 (NZ)
(72) Inventor: SCOTTI, Paul Douglas, Auckland (NZ); DEARING, Sally Caroline, Auckland (NZ); GREENWOOD, David Roger, Auckland (NZ); NEWCOMB, Richard, David, Auckland (NZ)
(74) Representative: Lee, Nicholas John
(86) International application number: PCT/NZ1999/000227
(87) International publication number: WO 2000/039165

(56) References cited:
- WO-A-96/12021
- SCOTTI PAUL D ET AL: "Pernin: A novel, self-aggregating haemolymph protein from the New Zealand green-lipped mussel, Perna canaliculus (Bivalvia: Mytilidae)." COMPARATIVE BIOCHEMISTRY AND PHYSIOLOGY PART B BIOCHEMISTRY &, vol. 128B, no. 4, April 2001 (2001-04), pages 767-779, XP002214786 April, 2001 ISSN: 1096-4959
- WEDDE MARIANNE ET AL: "Purification and characterization of an inducible metalloprotease inhibitor from the hemolymph of greater wax moth larvae, Galleria mellonella." EUROPEAN JOURNAL OF BIOCHEMISTRY, vol. 255, no. 3, 1 August 1998 (1998-08-01), pages 535-543, XP002214787 ISSN: 0014-2956
- MOROHASHI A ET AL: "Brevetoxin B3, a New Brevetoxin Analog Isolated from the Greenshell Mussel Perna canaliculus Involved in Neurotoxic Shellfish Poisoning in New Zealand" TETRAHEDRON LETTERS, ELSEVIER SCIENCE PUBLISHERS, AMSTERDAM, NL, vol. 36, no. 49, 4 December 1995 (1995-12-04), pages 8995-8998, XP004026792 ISSN: 0040-4039
- DATABASE STN MEDLINE ON-LINE 1999206055, February 1999 BARSYTE D. ET. AL.: 'Comparative Biochemistry and Physiology', XP002952821 & BARSYTE D. ET. AL. PART, C. PHARMACOLOGY, TOXICOLOGY AND ENDOCRINOLOGY vol. 122, no. 2, February 1999, pages 287 - 296
- CHEMICAL ABSTRACTS, 08 December 1998, Columbus, Ohio, US; abstract no. 130:48310, 'Sequence and applications in oyster larva settlement of marine Me1A gene.' XP001094823 & US 5 846 531 A (WEINER R.) 08 December 1998
- DATABASE STN MEDLINE ON-LINE 862200228, March 1986 ROESIJADI G., XP002952822 & ROESIJADI G. ENIVRONEMNTAL HEALTH PERSEPCTIVES vol. 65, March 1986, pages 45 - 48
- DATABASE STN MEDLINE ON-LINE PMID: 2861005, UI: 85229334 XP002952823 & FRAZIER JM. ET. AL. COMP. BIOCHEM. PHYSIOL. C. vol. 80, no. 2, 1985, pages 257 - 262
- CHEMICAL ABSTRACTS, vol. 67C, no. 2, 1980, Columbus, Ohio, US; abstract no. 94:62091, COMP. BIOCHEM. PHYSIOL. C.: 'Rapid induction of copper-binding proteins in the gills of metal exposed mussels.' page 215-218; XP002952824
- DATABASE WPI Week 198147, Derwent Publications Ltd., London, GB; Class B04, AN 1981-87230D, XP002952825 & WO 81 03124 A (PHARMACIA AB) 12 November 1981
- DATABASE WPI Week 199420, Derwent Publications Ltd., London, GB; Class B04, AN 1994-163935, XP002952826 SUNTORY LTD. & JP 06 107 682 A 19 April 1994
- DATABASE WPI Week 199420, Derwent Publications Ltd., London, GB; Class B04, AN 1994-163936, XP002952827 & JP 06 107 683 A (SUNTORY LTD.) 19 April 1994
- DATABASE WPI Derwent Publications Ltd., London, GB; Class D13, AN 1994-79466D, XP002952828 & US 4 293 098 A (SYSTEM CONSULTANS) 06 October 1981

### SEQUENCE LISTING

<110> The Horticulture and Food Research Institute of Ne
<120> Serine Protease Inhibitor
<130> 25409 MRB
<140>
   <141>
<150> NZ 336906
   <151> 1999-07-23
<160> 8
<170> PatentIn Ver. 2.1
<210> 1
   <211> 10
   <212> PRT
   <213> Perna canaliculus
<400> 1
   Asp Gly Glu Gln Cys Asn Asp Gly Gln Asn
    1               5                   10
<210> 2
   <211> 19
   <212> PRT
   <213> Perna canaliculus
<400> 2
   Gln Gly Gly His Glu Val Glu Ser Glu Arg Val Ala Cys Cys Val Ile
    1               5                  10                  15
   Gly Arg Ala
<210> 3
   <211> 9
   <212> PRT
   <213> Perna canaliculus
<400> 3
   Gly Gln Ser His Pro Glu Ile Val His
   1               5
<210> 4
   <211> 7
   <212> PRT
   <213> Perna canaliculus
<400> 4
   Tyr His Gly His Asp Asp Ala
   1               5
<210> 5
   <211> 7
   <212> PRT
   <213> Perna canaliculus
<400> 5
   Val Val Asn Glu Val His His
   1               5
<210> 6
   <211> 1491
   <212> DNA
   <213> Perna canaliculus
<220>
   <221> CDS
   <222> (1)..(1491)
<400> 6
   gay ggg gag cag tgt aac gat ggg cag aac aaa gat gac cac cat gac   48
   Asp Gly Glu Gln Cys Asn Asp Gly Gln Asn Lys Asp Asp His His Asp
     1               5                  10                  15
   gac cac cac gat gat cac cat gac gac cat gat gat gat gat gaa aca   96
   Asp His His Asp Asp His His Asp Asp His Asp Asp Asp Asp Glu Thr
                20                  25                  30
   atg cac tat gcc cag tgt gaa atg gaa cca aac cct cat atg gct agc   144
   Met His Tyr Ala Gln Cys Glu Met Glu Pro Asn Pro His Met Ala Ser
   35 40 45
   agc ctt cac cac cat gtc cat ggc agc ata gag ttg tca cag aag ggt 192
   Ser Leu His His His Val His Gly Ser Ile Glu Leu Ser Gln Lys Gly
   50 55 60
   cat gga gct gtt tat cta gaa ctt cat ctt gtc gga ttc aac aca agt 240
   His Gly Ala Val Tyr Leu Glu Leu His Leu Val Gly Phe Asn Thr Ser
   65 70 75 80
   gaa gac cat gac gac cac cat cat gga ctt cat ctg cac atg ctt ggt 288
   Glu Asp His Asp Asp His His His Gly Leu His Leu His Met Leu Gly
   85 90 95
   gac atg tca gca ggt tgt gat tct att ggc gaa ctg tac aat gct cac 336
   Asp Met Ser Ala Gly Cys Asp Ser Ile Gly Glu Leu Tyr Asn Ala His
   100 105 110
   cca gaa aaa cat gct gac cct ggt gac ctc ggt gac ctg gtt gac gat 384
   Pro Glu Lys His Ala Asp Pro Gly Asp Leu Gly Asp Leu Val Asp Asp
   115 120 125
   gat agg ggc gtg gtt aat gaa gtt cat cat tat gct tgg ttg gac att 432
   Asp Arg Gly Val Val Asn Glu Val His His Tyr Ala Trp Leu Asp Ile
   130 135 140
   gat ggt aca gca cca aac acc gaa gct ctc att gga cac tca atg act 480
   Asp Gly Thr Ala Pro Asn Thr Glu Ala Leu Ile Gly His Ser Met Thr
   145 150 155 160
   att tta caa ggg agt cac acc gat gct gat acc cca gcc agt aga atc 528
   Ile Leu Gln Gly Ser His Thr Asp Ala Asp Thr Pro Ala Ser Arg Ile
   165 170 175
   gcc tgt tgt gtt att ggt cat gga aaa gct cgc cca gaa aca gca gct 576
   Ala Cys Cys Val Ile Gly His Gly Lys Ala Arg Pro Glu Thr Ala Ala
   180 185 190
   gct cta cat cac gag cta gag gaa gat aaa act gag cat tat gcc cat 624
   Ala Leu His His Glu Leu Glu Glu Asp Lys Thr Glu His Tyr Ala His
   195 200 205
   tgt gac gta aga tct aat aca cac caa cca aag gct ctt cat cat cat 672
   Cys Asp Val Arg Ser Asn Thr His Gln Pro Lys Ala Leu His His His
   210 215 220
   gtc cac gga acc atc gat ttc aaa caa gtt ggt tat ggt gac ctt gaa 720
   Val His Gly Thr Ile Asp Phe Lys Gln Val Gly Tyr Gly Asp Leu Glu
   225 230 235 240
   gtg tcc tac cat tta gag gga ttt aat gta agt gat gac cac aaa gat 768
   Val Ser Tyr His Leu Glu Gly Phe Asn Val Ser Asp Asp His Lys Asp
   245 250 255
   cat ctc cat gac gta cag atc tac gcc aac ggt gac ctg acc agt gga 816
   His Leu His Asp Val Gln Ile Tyr Ala Asn Gly Asp Leu Thr Ser Gly
   260 265 270
   tgt gat aac ctc ggt gct aaa tat gat cct cat gaa gat tac cac agt 864
   Cys Asp Asn Leu Gly Ala Lys Tyr Asp Pro His Glu Asp Tyr His Ser
   275 280 285
   gag ttg ggt gat cta gga gat att cac gat gat gac cat ggc gtt gtc 912
   Glu Leu Gly Asp Leu Gly Asp Ile His Asp Asp Asp His Gly Val Val
   290 295 300
   aat gaa agc cac aga tat tcc tgg atc aat atc ttc ggt gat gac agt 960
   Asn Glu Ser His Arg Tyr Ser Trp Ile Asn Ile Phe Gly Asp Asp Ser
   305 310 315 320
   gtc ctg gga cgt tct att gcc att cac caa aga gac cat ctt cat aaa 1008
   Val Leu Gly Arg Ser Ile Ala Ile His Gln Arg Asp His Leu His Lys
   325 330 335
   agt gcc aaa att gcc tgt tgt gtc ata gga cgt gga cag agc cat cca 1056
   Ser Ala Lys Ile Ala Cys Cys Val Ile Gly Arg Gly Gln Ser His Pro
   340 345 350
   gaa att gtt cac aga gct aaa tgt gtt gtc aga cct aat aca gaa tct 1104
   Glu Ile Val His Arg Ala Lys Cys Val Val Arg Pro Asn Thr Glu Ser
   355 360 365
   act ggt tta cat cac cat gtc tct ggt tct ata aca ttc gaa cag acc 1152
   Thr Gly Leu His His His Val Ser Gly Ser Ile Thr Phe Glu Gln Thr
   370 375 380
   cct gga gga tca aca cat atg acg gct gat ctc aaa gga ttt aac gtt 1200
   Pro Gly Gly Ser Thr His Met Thr Ala Asp Leu Lys Gly Phe Asn Val
   385 390 395 400
   agt gag gac ttg tca cat cat cgt cat ggt gtg cag ctc cat gaa tgg 1248
   Ser Glu Asp Leu Ser His His Arg His Gly Val Gln Leu His Glu Trp
   405 410 415
   gga gat atg tcc cat ggc tgt cac tcc tta ggc aga atg tac cat ggt 1296
   Gly Asp Met Ser His Gly Cys His Ser Leu Gly Arg Met Tyr His Gly
                   420                      425                      430
   cat gat gat gct cat gac ccc aaa aga cct ggt gac ctt ggt gat gtt     1344
   His Asp Asp Ala His Asp Pro Lys Arg Pro Gly Asp Leu Gly Asp Val
           435                     440                     445
   ata gat gat tcc cat ggc atc gtt cat tca act aga acc ttt gat cat     1392
   Ile Asp Asp Ser His Gly Ile Val His Ser Thr Arg Thr Phe Asp His
       450                     455                     460
   ctt aat gtt gaa gat ctt aac gca cgt tcc ctt gtg att atg cag ggc     1440
   Leu Asn Val Glu Asp Leu Asn Ala Arg Ser Leu Val Ile Met Gln Gly
   465                     470                     475                     480
   gga cat gag gtc gag agt gag agg gtt gct tgc tgt gtt ata gga cgg     1488
   Gly His Glu Val Glu Ser Glu Arg Val Ala Cys Cys Val Ile Gly Arg
                           485                     490                     495
   gca                                                                 1491
   Ala
<210> 7
   <211> 497
   <212> PRT
   <213> Perna canaliculus
<400> 7
   Asp Gly Glu Gln Cys Asn Asp Gly Gln Asn Lys Asp Asp His His Asp
   1 5 10 15
   Asp His His Asp Asp His His Asp Asp His Asp Asp Asp Asp Glu Thr
   20 25 30
   Met His Tyr Ala Gln Cys Glu Met Glu Pro Asn Pro His Met Ala Ser
   35 40 45
   Ser Leu His His His Val His Gly Ser Ile Glu Leu Ser Gln Lys Gly
   50 55 60
   His Gly Ala Val Tyr Leu Glu Leu His Leu Val Gly Phe Asn Thr Ser
   65 70 75 80
   Glu Asp His Asp Asp His His His Gly Leu His Leu His Met Leu Gly
   85 90 95
   Asp Met Ser Ala Gly Cys Asp Ser Ile Gly Glu Leu Tyr Asn Ala His
   100 105 110
   Pro Glu Lys His Ala Asp Pro Gly Asp Leu Gly Asp Leu Val Asp Asp
   115 120 125
   Asp Arg Gly Val Val Asn Glu Val His His Tyr Ala Trp Leu Asp Ile
   130 135 140
   Asp Gly Thr Ala Pro Asn Thr Glu Ala Leu Ile Gly His Ser Met Thr
   145 150 155 160
   Ile Leu Gln Gly Ser His Thr Asp Ala Asp Thr Pro Ala Ser Arg Ile
   165 170 175
   Ala Cys Cys Val Ile Gly His Gly Lys Ala Arg Pro Glu Thr Ala Ala
   180 185 190
   Ala Leu His His Glu Leu Glu Glu Asp Lys Thr Glu His Tyr Ala His
   195 200 205
   Cys Asp Val Arg Ser Asn Thr His Gln Pro Lys Ala Leu His His His
   210 215 220
   Val His Gly Thr Ile Asp Phe Lys Gln Val Gly Tyr Gly Asp Leu Glu
   225 230 235 240
   Val Ser Tyr His Leu Glu Gly Phe Asn Val Ser Asp Asp His Lys Asp
   245 250 255
   His Leu His Asp Val Gln Ile Tyr Ala Asn Gly Asp Leu Thr Ser Gly
   260 265 270
   Cys Asp Asn Leu Gly Ala Lys Tyr Asp Pro His Glu Asp Tyr His Ser
   275 280 285
   Glu Leu Gly Asp Leu Gly Asp Ile His Asp Asp Asp His Gly Val Val
   290 295 300
   Asn Glu Ser His Arg Tyr Ser Trp Ile Asn Ile Phe Gly Asp Asp Ser
   305 310 315 320
   Val Leu Gly Arg Ser Ile Ala Ile His Gln Arg Asp His Leu His Lys
   325 330 335
   Ser Ala Lys Ile Ala Cys Cys Val Ile Gly Arg Gly Gln Ser His Pro
   340 345 350
   Glu Ile Val His Arg Ala Lys Cys Val Val Arg Pro Asn Thr Glu Ser
   355 360 365
   Thr Gly Leu His His His Val Ser Gly Ser Ile Thr Phe Glu Gln Thr
   370 375 380
   Pro Gly Gly Ser Thr His Met Thr Ala Asp Leu Lys Gly Phe Asn Val
   385 390 395 400
   Ser Glu Asp Leu Ser His His Arg His Gly Val Gln Leu His Glu Trp
   405 410 415
   Gly Asp Met Ser His Gly Cys His Ser Leu Gly Arg Met Tyr His Gly
   420 425 430
   His Asp Asp Ala His Asp Pro Lys Arg Pro Gly Asp Leu Gly Asp Val
   435 440 445
   Ile Asp Asp Ser His Gly Ile Val His Ser Thr Arg Thr Phe Asp His
   450 455 460
   Leu Asn Val Glu Asp Leu Asn Ala Arg Ser Leu Val Ile Met Gln Gly
   465 470 475 480
   Gly His Glu Val Glu Ser Glu Arg Val Ala Cys Cys Val Ile Gly Arg
   485 490 495
   Ala
<210> 8
   <211> 1611
   <212> DNA
   <213> Perna canaliculus
<220>
   <221> polyA_signal
   <222> (1557)..(1563)
<220>
   <221> misc_feature
   <222> (1492)..(1494)
   <223> Opal stop codon
<400> 8
   gayggggagc agtgtaacga tgggcagaac aaagatgacc accatgacga ccaccacgat 60
   gatcaccatg acgaccatga tgatgatgat gaaacaatgc actatgccca gtgtgaaatg 120
   gaaccaaacc ctcatatggc tagcagcctt caccaccatg tccatggcag catagagttg 180
   tcacagaagg gtcatggagc tgtttatcta gaacttcatc ttgtcggatt caacacaagt 240
   gaagaccatg acgaccacca tcatggactt catctgcaca tgcttggtga catgtcagca 300
   ggttgtgatt ctattggcga actgtacaat gctcacccag aaaaacatgc tgaccctggt 360
   gacctcggtg acctggttga cgatgatagg ggcgtggtta atgaagttca tcattatgct 420
   tggttggaca ttgatggtac agcaccaaac accgaagctc tcattggaca ctcaatgact 480
   attttacaag ggagtcacac cgatgctgat accccagcca gtagaatcgc ctgttgtgtt 540
   attggtcatg gaaaagctcg cccagaaaca gcagctgctc tacatcacga gctagaggaa 600
   gataaaactg agcattatgc ccattgtgac gtaagatcta atacacacca accaaaggct 660
   cttcatcatc atgtccacgg aaccatcgat ttcaaacaag ttggttatgg tgaccttgaa 720
   gtgtcctacc atttagaggg atttaatgta agtgatgacc acaaagatca tctccatgac 780
   gtacagatct acgccaacgg tgacctgacc agtggatgtg ataacctcgg tgctaaatat 840
   gatcctcatg aagattacca cagtgagttg ggtgatctag gagatattca cgatgatgac 900
   catggcgttg tcaatgaaag ccacagatat tcctggatca atatcttcgg tgatgacagt 960
   gtcctgggac gttctattgc cattcaccaa agagaccatc ttcataaaag tgccaaaatt 1020
   gcctgttgtg tcataggacg tggacagagc catccagaaa ttgttcacag agctaaatgt 1080
   gttgtcagac ctaatacaga atctactggt ttacatcacc atgtctctgg ttctataaca 1140
   ttcgaacaga cccctggagg atcaacacat atgacggctg atctcaaagg atttaacgtt 1200
   agtgaggact tgtcacatca tcgtcatggt gtgcagctcc atgaatgggg agatatgtcc 1260
   catggctgtc actccttagg cagaatgtac catggtcatg atgatgctca tgaccccaaa 1320
   agacctggtg accttggtga tgttatagat gattcccatg gcatcgttca ttcaactaga 1380
   acctttgatc atcttaatgt tgaagatctt aacgcacgtt cccttgtgat tatgcagggc 1440
   ggacatgagg tcgagagtga gagggttgct tgctgtgtta taggacgggc atgaataacc 1500
   tcactagagt gactttgtct aacatgacaa ttaacaattg tataacttcg ctaaaaaata 1560
   aaacaatgac acaatgnaaa aaaaaaaaaa aaaaaaaaaa aaaaaaaaaa a          1611

## Description

This invention relates to a protein and compositions which contain it. More particularly, it relates to a protein which *inter alia* exhibits activity as a metal cation binding agent and as an anti-thrombin agent.

### BACKGROUND

Thrombin is a serine protease involved in blood coagulation. It has specificity for the cleavage of arginine-lysine bonds as well as cleaving an arginine-threonine bond in pro-thrombin, releasing pre-thrombin which is subsequently cleaved to produce active thrombin. This active thrombin can then release more thrombin from pro-thrombin. In blood clotting and coagulation, thrombin cleaves fibrinopeptide B from fibrinogen as well as converting blood factors IX to IXa, V to Va, VIII to VIIIa and XIII to XIIIa.

Inhibitors of thrombin therefore inhibit coagulation and have application in any procedure where coagulation is undesirable. One such application is in the collection and storage of blood products. Another is in medicaments for preventing or reducing coagulation for example in treating or preventing cardiac malfunctions.

Anti-thrombin agents are known. One example is anti-thrombin III (AT-III). However, AT-III is capable of effectively inhibiting thrombin only in the presence of heparin.

The applicants have now identified a novel protein which has a range of activities, including anti-thrombin activity, and which when active against thrombin does not require heparin as a cofactor. It is towards this protein that the present invention is broadly directed.

### SUMMARY OF THE INVENTION

In a first aspect, the present invention provides an isolated protein which has a molecular weight of about 55 kDa and an amino acid sequence which includes one or more of the following:
(a) DGEQCNDGQN (SEQ ID NO. 1) ;
(b) QGGHEVESERVACCVIGRA (SEQ ID NO. 2) ;
(c) GQSHPEIVH (SEQ ID NO. 3) ;
(d) YHGHDDA (SEQ ID NO. 4) ;
(e) WNEVHH (SEQ ID NO. 5) ;
or an active fragment thereof.

In a further aspect, the invention provides an isolated protein which comprises the amino acid sequence of or an active fragment thereof.

In yet a further aspect, the invention provides an isolated protein which is obtainable from the haemolymph of *Perna canaliculus* which has an apparent molecular weight of 75 kda determined by PAGE and which has activity as a serine protease inhibitor and/or a divalent cation binding agent, or an active fragment thereof.

Conveniently, said protein or fragment has activity as:
(i) a serine protease inhibitor; or
(ii) a divalent cation binding agent.

The invention further provides a protein which is obtainable from *Perna canaliculus* or a shellfish other than *Perna canaliculus* and is a functionally equivalent variant of a protein or fragment as described above in that it is immunologically cross-reactive with a protein or fragment as described above and has at least the same serine protease inhibitor and/or divalent cation binding activity as a protein or fragment as described above.

In another aspect, the invention provides a polynucleotide encoding a protein or fragment as defined above.

The polynucleotide may comprise the nucleotide sequence of or a variant thereof.

The invention also provides a polynucleotide which has the nucleotide sequence of SEQ ID NO:8, as well as a polynucleotide which hybridises under stringent conditions to a polynucleotide as defined above.

Still further, the invention provides a vector or construct which includes a polynucleotide as defined above, as well as a host cell which expresses a polynucleotide as defined above.

In another aspect, the invention provides a composition which comprises a protein or fragment as defined above

The composition may be medicament, a food, a dietary supplement, (optionally including the protein or fragment associated with or bound to at least one divalent cation of dietary significance, such as calcium, magnesium or zinc) or a bioremediation agent.

### DESCRIPTION OF THE DRAWINGS

While the present invention is broadly as defined above, it also includes embodiments of which the following description provides examples. In particular, a better understanding of the present invention will be gained through reference to the accompanying drawings in which

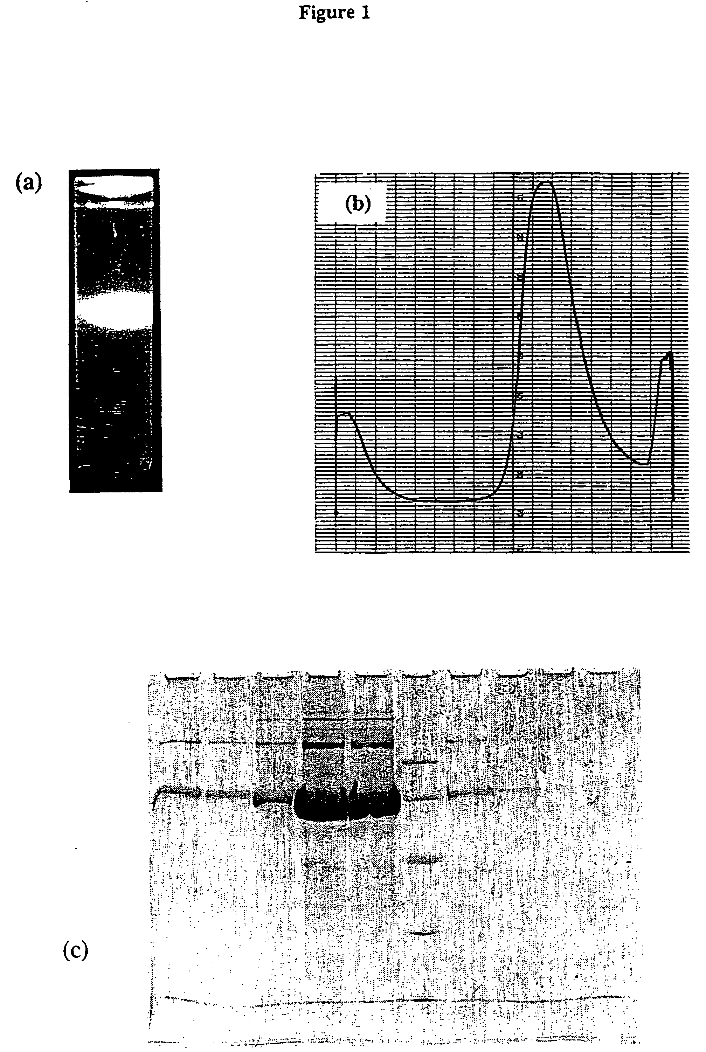

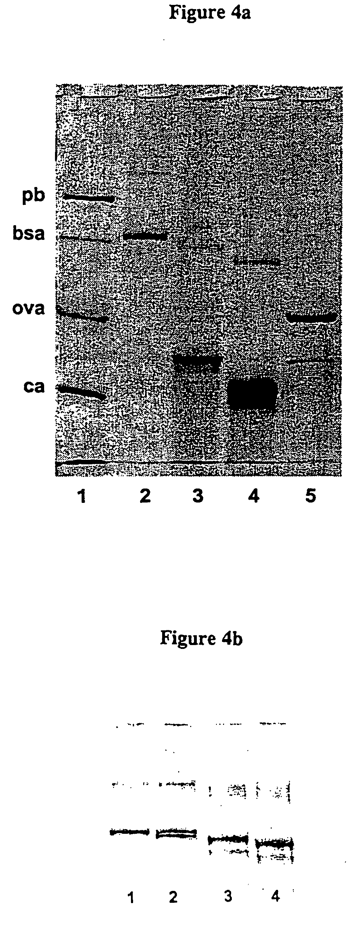

**Figure 1:** Purification of pernin from mussel haemolymph
**a)** light-scattering band following centrifugation of *P. canaliculus* haemolymph in CsCl; haemolymph was first centrifuged at low speed to remove haemocytes and then at high speed; the re-suspended pellet was then centrifuged in CsCl.
**b)** UV absorption profile (254 nm wavelength) from fractionation of the CsCl gradient; the light-scattering material in figure 1a appears as a peak.
**c)** protein composition in 1 ml fractions of a CsCl gradient following electrophoresis in a 12% polyacrylamide gel; the heavily stained (Coomassie) bands coincide with the position of the light-scattering and UV-absorbing regions of the gradient; the molecular weight was approximately 75 kDa as compared with polypeptide molecular weight standards (lane 6) (refer Figure 4a for standards). Lanes 1-5 and 7-9 contained samples from the CsCl gradient.

**Figure 2:** Virus-like particles observed by transmission electron microscopy of material in light scattering band in a CsCl gradient. Bar in micrograph represents 100 nm.

**Figure 3:** HPLC elution profile of pernin at 280 nm wavelength purified by CsCl gradient centrifugation..

**Figure 4:** SDS-PAGE profiles (12% gels) of aggregating protein species from *P. canaliculus* and other shellfish species
**a)** proteins extracted from whole shellfish and purified as described in Materials and Methods: lane 1: molecular weight standards (Bio-Rad, USA) :**pb** phosphorylase B, 97.4 kDa; **bsa** bovine serum albumin, 66 kDa; **ova** ovalbumin, 45 kDa; **ca** carbonic anhydrase, 31 kDa; lane 2: Greenshell™ mussel *P*. *canaliculus*; lane 3: blue mussel *Mytilis edulis*; lane 4: oyster *Crassostrea gigas*; lane 5: pipis *Paphies australis*.
**b)** PAGE analysis of human transferrin (Sigma, USA, MW *ca*. 80 kDa), a glycosylated protein, and pernin from *P. canaliculus* following treatment with endoglycosidase-F: lane 1: untreated transferrin; lane 2: transferrin treated with glycosidase-F; lane 3: untreated pernin lane 4: pernin treated with glycosidase-F.

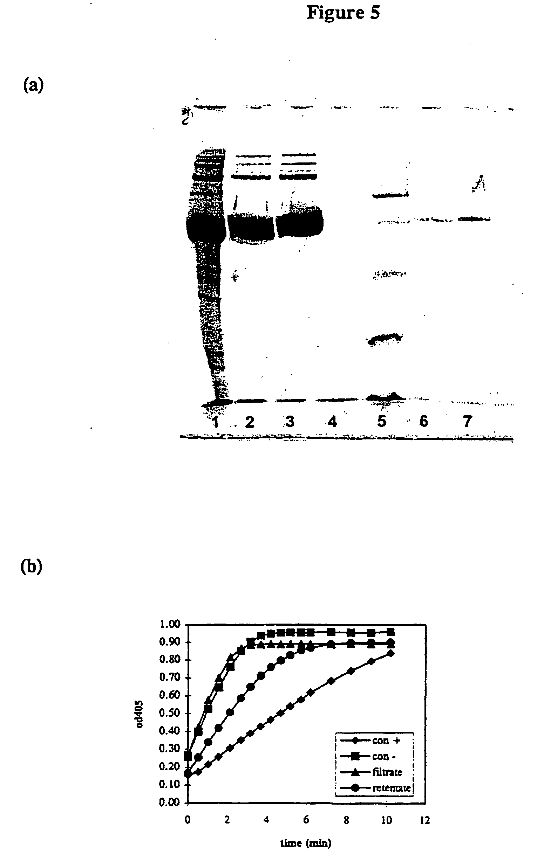

**Figure 5:** Activity of *P*. *canaliculus* haemolymph protein following centrifugation in a 30 kDa molecular weight exclusion filter for 10 min at 1000 *g* (Ultrafree-MC filter, 30,000 MW exclusion, Millipore, USA)
**a) SDS**-PAGE profile of haemolymph protein at various stages of purification. Lane 1: "crude" haemolymph (haemocytes removed); lane 2: resuspended pellet after ultracentrifugation of "crude" haemolymph for 80 min at 250,000 *g*, lane 3: pernin retentate; lane 4: filtrate (no proteins evident); lane 5: molecular weight markers, (refer Figure 4a); lanes 6,7: 10-fold dilutions of samples from lanes 2 and 3.
**b)** Anti-thrombin activity of 30,000 MW exclusion filter retentate and filtrate.
   **con+** = the standard 1/41 dilution of human plasma (i.e. standard anti-thrombin III activity);
   **con -** thrombin with no added plasma (buffer control); **filtrate:** material passed through a 30,000 MW exclusion filter; **retentate:** pernin protein retained by exclusion filter.

### DESCRIPTION OF THE INVENTION

As broadly outlined above, in one aspect the present invention provides a novel protein. The protein of the invention has an apparent molecular weight of 75 kDa, calculated by polyacrylamide gel electrophoresis (PAGE). The molecular weight inferred from the gene sequence is approximately 55 kDa.

One specific protein of the invention was initially identified as an extract from the New Zealand green lipped mussel *P*. *canaliculus*. It is therefore obtainable by extraction directly from *P*. *canaliculus*.

This protein has the amino acid sequence of SEQ ID NO. 7.

The protein of the invention can include its entire native amino acid sequence or can include only parts of that sequence where such parts constitute fragments which remain biologically active (active fragments). Such activity will normally be as a serine protease inhibitor or a divalent cation binding agent but is not restricted to these activities.

The invention also includes within its scope functionally equivalent variants of the protein of SEQ ID NO. 7.

The phrase "functionally equivalent variants" recognises that it is possible to vary the amino acid of a protein while retaining substantially equivalent functionality. For example, a protein can be considered a functional equivalent of another protein for a specific function if the equivalent peptide is immunologically cross-reactive with and has at least substantially the same function as the original protein.

The functionally equivalent protein need not be the same size as the original. The equivalent can be, for example, a fragment of the protein, a fusion of the protein with another protein or carrier, or a fusion of a fragment with additional amino acids. It is also possible to substitute amino acids in a sequence with equivalent amino acids using conventional techniques. Groups of amino acids normally held to be equivalent are:
(a) Ala, Ser, Thr, Pro, Gly;
(b) Asn, Asp, Glu, Gln;
(c) His, Arg, Lys;
(d) Met, Leu, Ile, Val; and
(e) Phe, Tyr, Trp.

Polypeptide sequences may be aligned, and percentage of identical amino acids in a specified region may be determined against another sequence, using computer algorithms that are publicly available. The similarity of polypeptide sequences may be examined using the BLASTP algorithm. BLASTP software is available on the NCBI anonymous FTP server (ftp://ncbi.nlm.nih.gov) under /blast/executables/. The use of the BLAST family of algorithms, including BLASTP, is described at NCBI's website at URL http://www.ncbi.nlm.nih.gov/BLAST/newblast.html and in the publication of Altschul, Stephen F., *et al*. (1997), "Gapped BLAST and PSI-BLAST: a new generation of protein database search programs", *Nucleic Acids Res.* 25:3389-34023.

The protein of the invention together with its active fragments and other variants may be generated by synthetic or recombinant means. Synthetic polypeptides having fewer than about 100 amino acids, and generally fewer than about 50 amino acids, may be generated by techniques well known to those of ordinary skill in the art. For example, such peptides may be synthesised using any of the commercially available solid-phase techniques such as the Merryfield solid phase synthesis method, where amino acids are sequentially added to a growing amino acid chain (see Merryfield, J. Am. Chem. Soc 85: 2146-2149 (1963)). Equipment for automative synthesis of peptides is commercially available from suppliers such as Perkin Elmer/Applied Biosystems, Inc. and may be operated according to the manufacturers instructions.

The protein, or a fragment or variant thereof, may also be produced recombinantly by inserting a polynucleotide (usually DNA) sequence that encodes the protein into an expression vector and expressing the protein in an appropriate host. Any of a variety of expression vectors known to those of ordinary skill in the art may be employed. Expression may be achieved in any appropriate host cell that has been transformed or transfected with an expression vector containing a DNA molecule which encodes the recombinant protein. Suitable host cells includes procaryotes, yeasts and higher eukaryotic cells. Preferably, the host cells employed are *E. coli*, yeasts or a mammalian cell line such as COS or CHO, or an insect cell line, such as SF9, using a baculovirus expression vector. The DNA sequence expressed in this matter may encode the naturally occurring protein, fragments of the naturally occurring protein or variants thereof.

DNA sequences encoding the protein or fragments may be obtained by screening an appropriate *P*. *canaliculus* cDNA or genomic DNA library for DNA sequences that hybridise to degenerate oligonucleotides derived from partial amino acid sequences of the protein. Suitable degenerate oligonucleotides may be designed and synthesised by standard techniques and the screen may be performed as described, for example, in Maniatis *et al*. Molecular Cloning - A Laboratory Manual, Cold Spring Harbour Laboratories, Cold Spring Harbour, NY (1989). The polymerase chain reaction (PCR) may be employed to isolate a nucleic acid probe from genomic DNA, a cDNA or genomic DNA library. The library screen may then be performed using the isolated probe.

Variants of the protein may be prepared using standard mutagenesis techniques such as oligonucleotide-directed site specific mutagenesis.

A specific polynucleotide of the invention has the nucleotide sequence of SEQ ID NO. 6 as follows:

A further polynucleotide has the sequence of SEQ ID NO. 8 as follows: with TGA being the opal stop codon and AATAAA the polyadenylation signal.

Variants or homologues of the above polynucleotide sequences also form part of the present invention. Polynucleotide sequences may be aligned, and percentage of identical nucleotides in a specified region may be determined against another sequence, using computer algorithms that are publicly available. Two exemplary algorithms for aligning and identifying the similarity of polynucleotide sequences are the BLASTN and FASTA algorithms. The BLASTN software is available on the NCBI anonymous FTP server (ftp://ncbi.nlm.nih.gov) under /blast/executables/. The BLASTN algorithm version 2.0.4 [Feb-24-1998], set to the default parameters described in the documentation and distributed with the algorithm, is preferred for use in the determination of variants according to the present invention. The use of the BLAST family of algorithms, including BLASTN, is described at NCBI's website at URL http://www.ncbi.nlm.nih.gov/BLAST/newblast.html and in the publication of Altschul, Stephen F, *et al* (1997). "Gapped BLAST and PSI-BLAST: a new generation of protein database search programs", *Nucleic Acids Res.* 25:3389-3402. The computer algorithm FASTA is available on the Internet at the ftp site ftp://ftp.virginia.edu.pub/fasta/. Version 2.0u4, February 1996, set to the default parameters described in the documentation and distributed with the algorithm, is preferred for use in the determination of variants according to the present invention. The use of the FASTA algorithm is described in the W R Pearson and D.J. Lipman, "Improved Tools for Biological Sequence Analysis," *Proc*. *Natl*. *Acad*. *Sci*. *USA 85*:2444-2448 (1988) and W.R. Pearson, "Rapid and Sensitive Sequence Comparison with FASTP and FASTA," *Methods in Enzymology 183*:63-98 (1990).

All sequences identified as above qualify as "variants" as that term is used herein.

Variant polynucleotide sequences will generally hybridize to the recited polynucleotide sequence under stringent conditions. As used herein, "stringent conditions" refers to prewashing in a solution of 6X SSC, 0.2% SDS; hybridizing at 65°C, 6X SSC, 0.2% SDS overnight; followed by two washes of 30 minutes each in 1X SSC, 0.1% SDS at 65°C and two washes of 30 minutes each in 0.2X SSC, 0.1% SDS at 65°C. Such hybridizable sequences include those which code for the equivalent protein from sources (such as shellfish) other than *P*. *canaliculus*.

While the above synthetic or recombinant approaches can be taken to produce the protein of the invention, it is however practicable (and indeed presently preferred) to obtain the protein by isolation from *P*. *canaliculus*. This reflects the applicants' finding that the protein is the dominant protein of the haemolymph of *P*. *canaliculus* and also that the protein is self-aggregating. It can therefore be isolated in commercially significant quantities direct from the mussel itself. For example, approximately 2 mg of the protein can be obtained per ml of haemolymph.

Once obtained, the protein is readily purified if desired. This will generally involve centrifugation in which the self-aggregating nature of the protein is important. Other approaches to purification (eg. chromatography) can however also be followed.

Furthermore, if viewed as desirable, additional purification steps can be employed using approaches which are standard in this art. These approaches are fully able to deliver a highly pure preparation of the protein.

Once obtained, the protein and/or its active fragments can be formulated into a composition. The composition can be, for example, a therapeutic composition for application as a pharmaceutical, or can be a health or dietary supplement. Again, standard approaches can be taken in formulating such compositions.

Still further, the composition can be a food in which the protein and/or its active fragments are included. This can occur by adding the protein to a pre-prepared foodstuff, or incorporating the protein into a step of the manufacturing process for the food.

The invention will now be described more fully in the following experimental section which is provided for illustrative purposes only.

### EXPERIMENTAL

### Section 1

### A. Materials and Methods

**A.1 Shellfish:** *Perna canaliculus* (the New Zealand green-lipped mussel; the Greenshell™ mussel) were obtained at retail supermarket outlets or from mussel farmers directly; other shellfish species were obtained from retail outlets except for the blue mussel *Mytilis edulis* which was supplied by Sanford's Fisheries (Havelock, New Zealand).
**A.2 Extracts:** Mussel extracts were prepared by homogenising whole, shucked mussels (up to 120 mm length) in a commercial food processor with the addition of 0.02 M sodium phosphate buffer, pH 7.2. Dichloromethane (1/2 volume) was mixed with the aqueous extract, centrifuged at low speed (6000 rpm, GSA rotor, Sorvall RC-5B centrifuge at 4 °C). Polyethylene glycol (PEG) (MW 6000) was added to the aqueous phase to a final concentration of 10% (w/v) and NaCl to 0.5 M and stirred at 4-6 °C overnight. Following low speed centrifugation the PEG-precipitate was resuspended in approximately 1/10 volume of sodium phosphate buffer. After another cycle of low-speed centrifugation the supernatant was centrifuged at high speed (50,000 rpm in a Beckman 60Ti rotor at 4 °C for 60-80 minutes). The resultant pellet was resuspended in a small volume of phosphate buffer and clarified by low speed centrifugation.
**A.3 Polyacrylamide gel electrophoresis:** 12% polyacrylamide gels (8 x10 cm; 1 mm thick) were cast using a prepared stock solution according to the manufacturer's instructions (40% acrylamide/bis solution 37.5:1, Bio-Rad, USA); commercially available 12% gels (Bio-Rad, USA) were also used. Samples (10 µl) were applied to lanes and the gels run at 160 V using a standard Tris/Glycine/SDS buffer (Bio-Rad, catalogue 161-0732) until the bromphenol blue marker reached the bottom of the gel. Gels were stained with BM Fast Stain Coomassie® (Boehringer Mannheim, Germany) and destained as per the manufacturer's instructions.
**A.4 Glycosylation test:** Samples were treated with N-glycosidase F (PNGase F from *Flavobacterium meningosepticum*; Boehringer Mannheim Biochemica, Germany) according to the manufacturer's directions. Treated and untreated samples were run in a standard 12% polyacrylamide gel.
**A.5 Isopycnic gradients:** CsCl (Boehringer Mannhein, Germany) solutions were prepared in 0.1 M sodium phosphate buffer, pH 7.2 and filtered through a 0.22 µm membrane (Acrodisc, Gelman Sciences, USA) to clarify. Two step gradients (1.25 g/cc top layer containing the sample and 1.45 g/cc bottom layer) were prepared as described by Scotti (1985) and centrifuged for approximately 17 hours at 20 °C in a Beckman 70Ti rotor at 30,000 rpm. The resultant gradient was fractionated by inserting a 100 µl glass capillary tube into the gradient and slowly pumping out the contents. UV absorbance was monitored by passing through a Uvicord spectrophotometer (LKB Produkter, Sweden). Fractions were collected and the refractive indices measured using an Abbé refractometer (Bellingham and Stanley, UK) and the density estimated using regression equations according to the method of Scotti (1985).
**A.6 Porous glass chromatography:** Controlled pore glass (CPG 240-80, Sigma Chemical Co., USA) was treated according to the suppliers directions. A 1 cm x 100 cm column (Bio-Rad, USA) was prepared. Samples (1-2 ml) were loaded onto the column and eluted with 0.1 M sodium phosphate buffer, pH 7.2, through a Uvicord spectrophotometer, fractions being collected at regular intervals.
**A.7 Estimation of protein concentration:** Concentrations were estimated using a bovine serum albumin standard (Blot Qualified BSA, Promega, USA) by UV absorption according to the method of Layne (1957) using the equation: mg/ml protein = 1.55*A₂₈₀ - 0.76*A₂₆₀. Alternatively, concentration was estimated by the Bradford reaction using reagent supplied by Bio-Rad (USA) at a wavelength of 620 nm..
A.8 High performance liquid chromatography: Reversed-phase HPLC was performed on an HP 1050 Ti-series HPLC (Hewlett Packard, USA) fitted with an analytical 300 ÅVydac C-18 column, 25 cm x 4.6 mm i.d.. The 10 µl sample in water was eluted with a 0-100% acetonitrile in water (v/v) gradient over 60 min and the absorption at 218 and 280 nm was recorded.

### B. Results

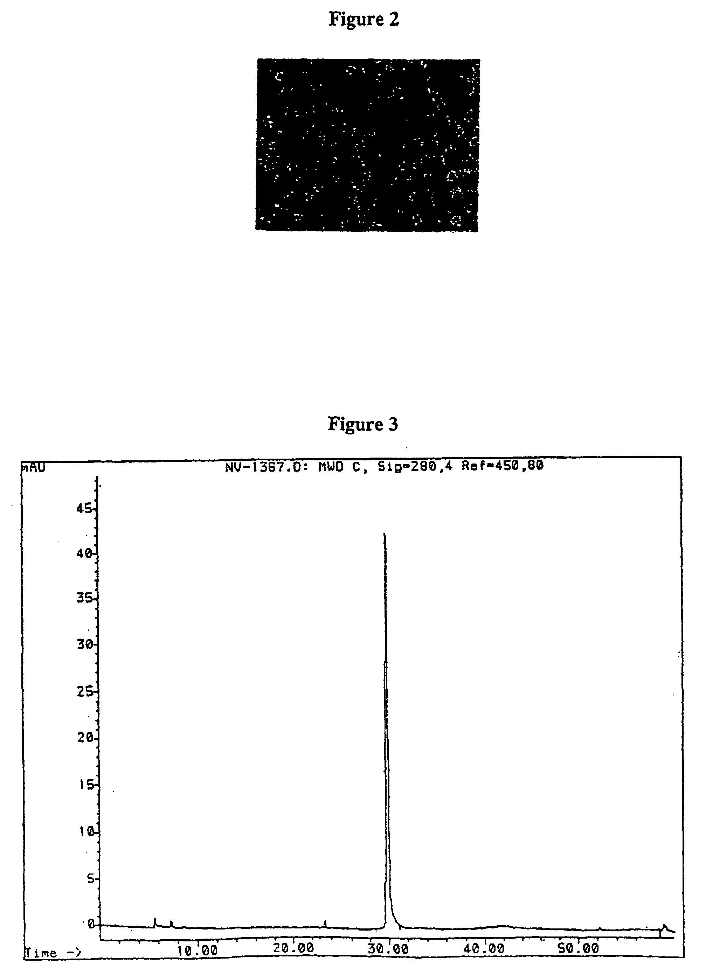

A light-scattering band was seen after centrifugation of extracts of whole Greenshell™ mussels in CsCl gradients **(Figures 1a and 1b).** The density of this band was estimated at 1.368 g/cc. A minor band was sometimes observed at approximately 1.390 g/cc. If rebanded in CsCl the 1.390 band yielded two bands - one at 1.390 g/cc and a second at 1.368 g/cc. SDS-PAGE analysis of fractions of either density gave similar polypeptide profiles with a single major band. The molecular weight of the protein by PAGE was estimated as 75,000 (75 kDa) **(Figure 1c).** Several minor bands of higher molecular weight and an additional minor band of 45 kDa were also seen. The main band (called pernin) at 75 kDa was always at great excess compared to the minor bands. When material from the light-scattering material from CsCl gradients were examined by electron microscopy, particles resembling those of "empty" small RNA viruses were seen **(Figure 2).** However a UV wavelength scan (data not shown) indicated that little, if any, nucleic acid was present and that the particles were mainly composed of protein. HPLC showed the CsCl band to be composed almost solely of a single species of protein **(Figure 3).** Since HPLC indicated a high degree of purity, the higher molecular weight polypeptides are presumed to be multimers of pernin. It is likely that the minor, lower molecular weight band is degraded pernin.

Chromatography, on a CPG 240-80 column, of semi-purified extracts, or of material banded in CsCl, showed that the majority of pernin was eluted in the exclusion volume using low molarity phosphate or Tris buffer as the eluent. In contrast, a protein of similar size, bovine serum albumin (68 kDa), was included in the column matrix. It appears, therefore, that pernin does aggregate into large, particle-like structures under certain conditions as suspected from the particles seen in **Figure 2**. HPLC confirmed that pernin from *P*. *canaliculus* obtained by CPG chromatography was highly purified. Aggregating protein species were also detected in extracts of other shellfish: the blue mussel *Mytilis edulis*, the oyster *Crassostrea gigas*, and New Zealand pipis *Paphies australis* but not in scallops *Pecten novaezealandiae.* These polypeptides were lower in molecular weight than pernin **(Figure 4a).** The pernin from *P*. *canaliculus* is N-glycosylated as shown by a reduction in molecular weight when treated with endoglycosidase-F before PAGE **(Figure 4b).**

The yield of pernin from whole mussel extractions averaged about 200 µg/mussel. Improved yields of pernin were obtained by extracting haemolymph directly from live *P*. *canaliculus*. A small notch was made in the shell using a triangular file and a 30 gauge needle inserted into the posterior adductor muscle. From 1 to 5 ml of haemolymph can be withdrawn easily. The haemolymph was spun at low speed (≈1000 *g*) to remove haemocytes and the resulting supernatant processed by ultracentrifugation, for example at 250,000 *g* for 40 minutes, followed by either CPG chromatography eluting with 0.1 M sodium phosphate buffer, pH 7.2, or isopycnic banding in CsCl in phosphate buffer. The pernin obtained in this way appeared no different than that purified from whole mussels and had the advantage of a 30-fold average increase in yield from each mussel. Haemolymph contained around 2 mg/ml (average ≈5-6 mg/mussel) of pernin which is by far the most predominant polypeptide species **(Figure 5a).** The time to purify pernin was reduced from about 5 days to 1 day.

Microsequencing of the N-terminal region and internal fragments generated by chemical and enzymatic cleavage from purified pernin was performed and generated the following sequences of cleavage fragments:
(a) DGEQCNDGQN
(b) QGGHEVESERVACCVIGRA
(c) GQSHPEIVH
(d) YHGHDDA
(e) VVNEVHH.

These sequences code for amino acids as follows:

### CODE:

- A: alanine
- C: cystine
- D: aspartic acid
- E: glutamic acid
- F: phenylalanine
- G: glycine
- H: histidine
- I: isoleucine
- K: lysine
- L: leucine
- M: methionine
- N: asparagine
- P: proline
- Q: glutamine
- R: arginine
- S: serine
- T: threonine
- V: valine
- W: tryptophan
- Y: tyrosine

The sequence data was then compared with amino acid sequences in searchable computer data bases. Some sequences were found to be of particular interest:
**a)** a 10 amino acid residue sequence from the N-terminus of pernin (sequence (a) above) showed only homology with an 8 base anti-thrombin protein sequence from terrestrial leeches (data from US Patent 5,455,181 Oct 3, 1995: sequence 10). 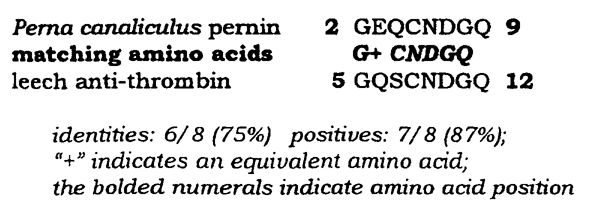
**b)** An internal cleavage product (sequence (b) above) was shown to be have homology to the Cu-Zn class of proteins known as "SODs" (superoxide dismutases).

Each of fragments (a) to (e) are part of the larger pernin amino acid sequence:

(Bold characters indicate directly sequenced fragments (a) to (e)).

### Section 2

### Anti-thrombin Activity

The possibility that pernin could function as an anti-thrombin agent was examined in a kinetic assay for thrombin inhibition.

### Thrombin inhibition assay

Kinetic assays were done using an Accucolor™ Antithrombin III kit (catalogue no. CRS105, Sigma Diagnostics, USA) with the reagents prepared according to the supplier's directions. Standard plasma was supplied by Instrumentation Laboratories (Italy) and used at the recommended dilution of 1/41. Samples of purified mussel protein in water were diluted 9/10 by adding 10X Sigma sample buffer. Heparin was purchased from Instrumentation Laboratories. Thrombin activity was estimated colorimetrically at 405 nm using a chromogenic substrate (H-D-HHT-L-Ala-L-Arg-pNa.2AcOH, catalogue no. A 8058, Sigma, USA) and a Multiskan Biochromatic plate reader (Labsystems, Finland)

This verified that pernin had inhibitory activity. When a purified preparation of pernin was centrifuged through a 30,000 MW exclusion filter **(Figure 5a)**, all the anti-thrombin activity was in the retentate and no detectable activity was present in the filtrate **(Figure 5b).** The standard serum was diluted 1/41 as recommended for this assay system; the pernin concentration was not determined directly but was in the 1 mg/ml range. From this kinetic data pernin inhibition was estimated to be about 50% of the level of human plasma (approximately 1 mg/ml pernin diluted 9/10 compared with the 1/41 plasma dilution in the standard ATIII assay system). Heparin, a co-factor required for ATIII inhibition of thrombin, was not required for inhibitory action by pernin.

### Metal Binding Activity

Hi Trap® Chelating affinity columns (Amersham Pharmacia Biotech, 1ml size) were prepared according to the manufacturer's instructions. The columns were then charged with either 0.1M cupric chloride or zinc chloride before equilibrating in a buffer (0.050M sodium phosphate and 0.5M sodium chloride containing 0.5mM imidazole, pH 7.0). Protein samples purified using CsCl centrifugation were suspended in this buffer and applied to the column using a chromatographic system (Econo System, Bio-Rad Laboratories, USA). Following washing of the column for 5 mins with buffer during which no protein appeared in the eluate, a linear gradient over 20 min at 1 ml/min was used to develop the column using buffer with the imidazole concentration at 100mM from 0-100%. The protein eluted into the gradient being retained longer on the copper chelation column than the zinc. The absorption of the eluate was monitored at 254nM.

Divalent metal ion content of the CsCl purified protein was determined by dissolving the protein in water at 10 mg/ml and analysing metal content by both atomic absorption and plasma emission spectrometry by comparison with a water blank. There was no significant divalent cation content in the protein purified by this method. However, purification by other methods not employing chaotropic agents like CsCl, the high content of histidine coupled with acidic amino acid residues and the likely origin of this protein from a SOD precursor, points to pernin having endogenous metal ions as part of its native structure.

### Section 3

### Gene Sequencing Method

A suite of non-specific primers called pUZ5 was synthesised by Gibco-BRL for the initial sequencing based on the N-terminal sequence of pernin. The general formula was:

Where Y represents a pyrimidine base, R represents a purine base and N represents any one of the four nucleotide bases. Sequencing was done, initially using pUZ5 and an oligo-dT based "bottom stand" primer from PCR amplified cDNA. Sequencing was done by dye-termination cycle sequencing using "BigDye" prism technology (Applied Biosystems Incorporated, USA) according to their instructions. Products were resolved on an ABI 377 automated sequencer. Following the initial sequencing of approximately 500 base pairs pernin-specific primers were constructed and used to complete the sequencing of the pernin gene.

This provided the following:

### Discussion

The present invention is a novel protein obtainable from *Perna canaliculus*, the New Zealand green-lipped (Greenshell™) mussel. The protein appears to be able to self-aggregate in structures resembling small virus like particles (VLPs) approximately 25 nm in diameter but lacking any nucleic acid. The protein was found in extracts of whole mussels and appears to be the predominant protein in haemolymph. The molecular weight of the protein was estimated to be 75 kDa by PAGE and inferred to be 55 kDa from its polynucleotide encoding sequence but, because of its ability to aggregate, the protein can be sedimented by ultracentrifugation in a short time (e.g. 40 minutes at 250,000 *g*) whereas the monomeric protein would not. Each ml of haemolymph yields, on the average, about 2 mg of pernin. Haemolymph is easily obtained by withdrawing fluid from the posterior adductor muscle of the shellfish which can yield up to 5 ml without obvious harm; it is not necessary to kill the mussel. The haemolymph obtained not only contains high levels of pernin but is quite free of contaminating materials, particularly compared with whole mussel extracts, so purification of pernin is simple. For highly pure preparations of pernin, ultracentrifugation is followed by isopycnic banding in a suitable density gradient medium such as CsCl.

The sequence of the N-terminus of pernin suggested that the protein might have anti-thrombin activity. This was demonstrated in kinetic assays on purified pernin. Since thrombin is a serine protease, pernin also acts as a serine protease inhibitor.

Comparison of the sequences obtained from several cleavage fragments against amino acid sequences in a computer database suggest that in addition to the anti-thrombin activity of pernin, the protein also possesses other activities. One of these is the ability to bind divalent cations such as Zn²⁺ and Cu²⁺.

### INDUSTRIAL APPLICATION

The preferred protein of the invention, pernin, has a number of utilities.

Because of its anti-thrombin activity pernin is potentially useful as an anticoagulant agent. Thrombin normally acts as a protease which converts fibrinogen in the blood to fibrin. Blood coagulation is counteracted by inhibitors, normally anti-thrombin III (ATIII); pernin has also been shown to inhibit thrombin activity in an ATIII assay system. In contrast to ATIII, whose action is accelerated by the presence of heparin (a sulphated mucopolysaccharide) pernin does not require heparin as a co-factor.

The pernin protein from *P*. *canaliculus* thus has value as a pharmaceutical. Since it is active as an anticoagulant in its native state it may also be useful as a natural therapeutic agent or health supplement. It is readily obtained as a natural product in high concentrations from mussel haemolymph. To obtain a highly pure preparation it is necessary only to remove haemocytes by centrifugation (or any other suitable method) followed by either ultracentrifugation (since pernin forms aggregates which readily sediment) and resuspension, isopycnic banding in a suitable medium such as CsCl, exclusion filtration through a suitable membrane which retains pernin, or chromatography through a medium such as controlled pore glass of suitable porosity. The result is a highly pure preparation of pernin.

The mussel *P*. *canaliculus* produces large amounts of the protein naturally, with little cost or effort involved in production, processing or purification.

A further utility of the protein arises from the fact that pernin can be stripped of divalent cations (for example by CsCl isopycnic banding, or pH variation). This allows for the addition of divalent cations of choice (such as Mg⁺⁺, Cd⁺⁺, Zn⁺⁺ or Ca⁺⁺) to the metal stripped pernin. Such a protein, with a modified and pre-selected divalent cation loading, has application in the food and nutraceutical industries.

The ability to bind divalent metal cations also gives rise to applications of the protein in bioremediation and/or cation recovery processes. The divalent cations can be present as contaminants or pollutants in, for example, a solution, and the solution passed by a substrate to which the protein is bound so that the cations are extracted.

Yet a further utility arises from the fact that the protein is "self-aggregating", and can form into structures resembling empty virus-like particles of approximately 25 nm in diameter. These empty virus-like particles are able to sequester other molecules inside them, with the consequent ability to function as delivery vehicles for those other molecules. Examples of molecules able to be delivered in this manner include pharmaceutically active compounds.

Those persons skilled in the art will understand that the above description is provided by way of illustration only and that the invention is limited only by the appended claims.

### REFERENCES

Layne, E. (1957). Spectrophotometric and turbidometric methods for measuring proteins, *Methods in Enzymology* **III,** 447.

Scotti, P.D. (1985). The estimation of virus density in isopycnic cesium chloride gradients. *Journal of Virological Methods* **12,** 149.

## Claims

1. An isolated protein which has a molecular weight of about 55 kDa and an amino acid sequence which includes one or more of the following:
(a) SEQ ID No. 1;
(b) SEQ ID No. 2;
(c) SEQ ID No. 3;
(d) SEQ ID No. 4;
(e) SEQ ID No. 5;
or an active fragment thereof.

2. An isolated protein which comprises the amino acid sequence of SEQ ID No. 7, or an active fragment thereof.

3. An isolated protein which is obtainable from the haemolymph of *Perna canaliculus* which has an apparent molecular weight of 75 kDa determined by PAGE and which has activity as a serine protease inhibitor and/or a divalent cation binding agent, or an active fragment thereof.

4. A protein or fragment as claimed in claim 1 or claim 2 which has activity as:
(i) a serine protease inhibitor; or
(ii) a divalent cation binding agent.

5. A protein which is obtainable from *Perna canaliculus* or a shellfish other than *Perna canaliculus* and is a functionally equivalent variant of a protein or fragment as claimed in claim 3 or claim 4 in that it is immunologically cross-reactive with a protein or fragment as claimed in claim 3 or claim 4 and has at least the same serine protease inhibitor and/or divalent cation binding activity as a protein or fragment as claimed in claim 3 or claim 4.

6. A polynucleotide encoding a protein or fragment as claimed in any one of claims 1 to 5.

7. A polynucleotide as claimed in claim 6 which comprises the nucleotide sequence of SEQ ID NO. 6 or a variant thereof.

8. A polynucleotide which has the nucleotide sequence of SEQ ID NO. 8.

9. A polynucleotide which hybridises under stringent conditions to a polynucleotide as claimed in any one of claims 6 to 8.

10. A vector which includes a polynucleotide as claimed in any one of claims 6 to 9.

11. A host cell which expresses a polynucleotide as claimed in any one of claims 6 to 9.

12. A composition which comprises a protein or fragment as claimed in any one of claims 1 to 5.

13. A composition as claimed in claim 12 which is a medicament, food or dietary supplement.

14. A dietary supplement as claimed in claim 13 in which said protein or fragment is associated with or bound to at least one divalent cation of dietary significance.

15. A dietary supplement as claimed in claim 14 wherein said divalent cation is calcium, magnesium or zinc.

16. A composition as claimed in claim 12 which is a bioremediation agent.

## Patentansprüche

1. Isoliertes Protein mit einem Molekulargewicht von ungefähr 55 kDa und einer Aminosäuresequenz, welche eine oder mehrere der folgenden Sequenzen aufweist:
(a) SEQ ID Nr. 1;
(b) SEQ ID Nr. 2;
(c) SEQ ID Nr. 3;
(d) SEQ ID Nr. 4;
(e) SEQ ID Nr. 5;
oder ein aktives Fragment davon.

2. Isoliertes Protein, welches die Aminosäuresequenz von SEQ ID Nr. 7 oder ein aktives Fragment davon enthält.

3. Isoliertes Protein, das aus der Hämolymphe der *Perna canaliculus* erhältlich ist, das ein durch PAGE bestimmtes scheinbares Molekulargewicht von 75 kDA und Aktivität als Serinproteasehemmer und/oder als zweiwertiger kationenbindender Wirkstoff aufweist, oder ein aktives Fragment davon.

4. Protein oder Fragment nach Anspruch 1 oder 2, welches Aktivität als
(i) Serinproteasehemmer oder
(ii) zweiwertiger kationenbindender Wirkstoff aufweist.

5. Protein, das aus *Perna canaliculus* oder einer anderen Muschel als *Perna canaliculus* erhältlich ist und insofern eine funktional äquivalente Variante eines Proteins oder Fragmentes nach Anspruch 3 oder 4 ist, als es mit einem Protein oder Fragment nach Anspruch 3 oder Anspruch 4 immunologisch kreuzreaktiv ist und mindestens denselben Serinproteasehemmer und/oder dieselbe zweiwertige kationenbindende Aktivität wie ein Protein oder Fragment nach Anspruch 3 oder Anspruch 4 aufweist.

6. Polynukleotid, das ein Protein oder ein Fragment nach einem der Ansprüche 1 bis 5 kodiert.

7. Polynukleotid nach Anspruch 6, welches die Nukleotidsequenz von SEQ ID Nr. 6 oder einer Variante davon aufweist.

8. Polynukleotid, welches die Nukleotidsequenz von SEQ ID Nr. 8 aufweist.

9. Polynukleotid, welches unter stringenten Bedingungen mit einem Polynukleotid nach einem der Ansprüche 6 bis 8 hybridisiert.

10. Vektor, welcher ein Polynukleotid nach einem der Ansprüche 6 bis 9 enthält.

11. Wirtszelle, welche ein Polynukleotid nach einem der Ansprüche 6 bis 9 exprimiert.

12. Zusammensetzung, welche ein Protein oder ein Fragment nach einem der Ansprüche 1 bis 5 enthält.

13. Zusammensetzung nach Anspruch 12, welche ein Medikament, Nahrungsmittelergänzungsmittel oder dietätisches Ergänzungsmittel ist.

14. Dietätisches Ergänzungsmittel nach Anspruch 13, wobei das Protein oder das Fragment mit mindestens einem zweiwertigen Kation von dietätischer Bedeutung assoziiert oder an ein solches gebunden ist.

15. Dietätisches Ergänzungsmittel nach Anspruch 14, wobei das zweiwertige Kation Kalzium, Magnesium oder Zink ist.

16. Zusammensetzung nach Anspruch 12, welche ein Bioremediations-Wirkstoff ist.

## Revendications

1. Protéine isolée qui présente un poids moléculaire d'environ 55 kDa et une séquence d'acides aminés qui comprend une ou plusieurs séquences parmi les suivantes :
(a) SEQ ID No. 1 ;
(b) SEQ ID No. 2 ;
(c) SEQ ID No. 3 ;
(d) SEQ ID No. 4 ;
(e) SEQ ID No. 5 ;
ou fragment actif de celle-ci.

2. Protéine isolée qui comprend la séquence d'acides aminés de la SEQ ID No. 7 ou un fragment actif de celle-ci.

3. Protéine isolée qui peut être obtenue à partir de l'hémolymphe de Perna canaliculus qui présente un poids moléculaire apparent de 75 kDa déterminé par PAGE et qui a l'activité d'un inhibiteur de sérine protéase et/ou d'un agent de liaison de cation divalent, ou fragment actif de celle-ci.

4. Protéine ou fragment selon la revendication 1 ou 2 qui présente l'activité
(i) d'un inhibiteur de sérine protéase ; ou
(ii) d'un agent de liaison de cation divalent.

5. Protéine qui peut être obtenue à partir de Perna canaliculus ou d'un crustacé autre que Perna canaliculus et qui est une variante fonctionnellement équivalente d'une protéine ou d'un fragment selon la revendication 3 ou 4 en ce qu'elle est présente une réactivité croisée sur le plan immunologique avec une protéine ou un fragment selon la revendication 3 ou 4 et présente au moins la même activité d'inhibiteur de sérine protéase et/ou d'agent de liaison de cation divalent que la protéine ou le fragment selon la revendication 3 ou 4.

6. Polynucléotide codant pour une protéine ou un fragment selon l'une quelconque des revendication 1 à 5.

7. Polynucléotide selon la revendication 6 qui comprend la séquence nucléotidique de la SEQ ID No. 6 ou une variante de celle-ci.

8. Polynucléotide qui a la séquence nucléotidique de la SEQ ID No. 8.

9. Polynucléotide qui réalise une hybridation dans des conditions strictes avec un polynucléotide selon l'une quelconque des revendications 6 à 8.

10. Vecteur qui comprend un polynucléotide selon l'une quelconque des revendications 6 à 9.

11. Cellule hôte qui exprime un polynucléotide selon l'une quelconque des revendications 6 à 9.

12. Composition qui comprend une protéine ou un fragment selon l'une quelconque des revendications 1 à 5.

13. Composition selon la revendication 12 qui est un médicament, un aliment ou un supplément diététique.

14. Supplément diététique selon la revendication 13 dans lequel ladite protéine ou ledit fragment est associé(e) ou lié(e) à au moins un cation divalent ayant une signification diététique.

15. Supplément diététique selon la revendication 14 dans lequel ledit cation divalent est du calcium, du magnésium ou du zinc.

16. Composition selon la revendication 12 qui est un agent de bioremédiation.
